# FASCICULE DE BREVET EUROPEEN

(11) **EP 1 993 574 B1**
(45) Date de publication et mention de la délivrance du brevet: **14.12.2011**
(21) Numéro de dépôt: 07726744.1
(22) Date de dépôt: 09.03.2007
(51) Int. Cl.: A61P 37/06, A61K 35/14, A61K 35/30, C12N 5/095

(54) **CELLULES T REGULATRICES CD4+CD25+ SPECIFIQUES POUR LA GREFFE DE CELLULES HEMATOPOÏETIQUES ET LA TOLERANCE IMMUNITAIRE**
SPEZIFISCHE CD4+CD25+-REGULATORISCHE T-ZELLEN FÜR DIE HÄMATOPOETISCHE ZELLTRANSPLANTATION UND IMMUNTOLERANZ
SPECIFIC CD4+CD25+ REGULATORY T CELLS FOR HAEMATOPOIETIC CELL TRANSPLANTATION AND IMMUNE TOLERANCE

(30) Priorité: 10.03.2006 FR 0650828
(43) Date de publication de la demande: 26.11.2008
(73) Titulaire: GENETHON, 91000 Evry (FR); Centre National de la Recherche Scientifique, 75794 Paris (FR)
(72) Inventeur: GROSS, David Alexandre, 94800 Vincennes (FR); DAVOUST, Jean, 78100 Saint Germain en Laye (FR)
(74) Mandataire: Buchet, Anne
(86) Numéro de dépôt international: PCT/EP2007/052222
(87) Numéro de publication internationale: WO 2007/104715

(56) Documents cités:
- EP-A- 1 241 249
- FR-A- 2 864 546
- GROSS DAVID-ALEXANDRE ET AL: "Simple conditioning with monospecific CD4+CD25+ regulatory T cells for bone marrow engraftment and tolerance to multiple gene products." BLOOD 15 SEP 2006, vol. 108, no. 6, 15 septembre 2006 (2006-09-15), pages 1841-1848, XP002408949 ISSN: 0006-4971
- JOFFRE OLIVIER ET AL: "Induction of antigen-specific tolerance to bone marrow allografts with CD4+CD25+ T lymphocytes" BLOOD, vol. 103, no. 11, 1 juin 2004 (2004-06-01), pages 4216-4221, XP002449596 ISSN: 0006-4971
- GROSS DAVID-ALEXANDRE ET AL: "CD4+CD25+ regulatory T cells inhibit immune-mediated transgene rejection." BLOOD, vol. 102, no. 13, 15 décembre 2003 (2003-12-15), pages 4326-4328, XP002408950 ISSN: 0006-4971
- MIKLOS DAVID B ET AL: "Antibody responses to H-Y minor histocompatibility antigens correlate with chronic graft-versus-host disease and disease remission" BLOOD, vol. 105, no. 7, avril 2005 (2005-04), pages 2973-2978, XP002408951 ISSN: 0006-4971
- VOGT MARIO H J ET AL: "The DBY gene codes for an HLA-DQ5-restricted human male-specific minor histocompatibility antigen involved in graft-versus-host disease" BLOOD, vol. 99, no. 8, 15 avril 2002 (2002-04-15), pages 3027-3032, XP002408952 ISSN: 0006-4971

## Description

L'invention concerne des cellules T régulatrices CD4+CD25+ spécifiques d'un antigène mineur, et plus précisément leur association avec des cellules souches hématopoïétiques dans une composition pharmaceutique, ainsi que leur utilisation comme médicament pour augmenter la tolérance immunitaire d'un hôte histocompatible, y compris vis-à-vis de transgènes.

La thérapie de remplacement de gènes a suscité un grand intérêt pour le traitement de différentes maladies métaboliques et de désordres génétiques tels que les dystrophies musculaires, ou l'hémophilie. Il est toutefois très important d'éviter des réponses immunes délétères. Des efforts considérables ont été faits pour améliorer les procédures de délivrance des gènes, en recherchant les vecteurs les moins immunogènes, des promoteurs tissu-spécifiques ainsi que des voies et doses d'injection appropriées (1, 2). Cependant ces approches sont confrontées au risque de créer un statut d'ignorance immune, dans lequel une inflammation secondaire ou les signaux de pathogènes de voisinage peuvent induire un rejet immun des produits de gène thérapeutique (3). L'induction de la tolérance périphérique en utilisant des co-traitements immunosuppressifs ou le blocage transitoire de voies de co-stimulation, développés initialement pour la transplantation ou les applications d'auto-immunité, ont été transposés avec succès à la thérapie génique (4, 5) mais ces procédures s'accompagnent d'une toxicité marquée, augmentant les risques de maladie cardiovasculaire, d'infections opportunistes et de tumeurs malignes chez l'homme. Des approches de tolérance spécifique vis-à-vis des transgènes sont nécessaires pour bloquer le rejet initial et pour induire une tolérance à long terme envers les transgènes, sans affecter les fonctions immunes générales des patients. Ces requis sont satisfaits en exprimant les transgènes d'intérêt dans le système hématopoïétique avant les interventions de thérapie génique.

Le chimérisme hématopoïétique mixte, avec la tolérance donneur-spécifique robuste qui lui est associée, peut être utilisé dans la transplantation d'organes (6) et le traitement des maladies autoimmunes (7). Le chimérisme hématopoïétique mixte a été utilisé récemment pour induire une tolérance envers des protéines spécifiques en transférant des gènes dans des cellules souches hématopoïétique (CSH) autologues pour la thérapie génique **(8).** Cependant, son induction stable nécessite une immunodépression de longue durée et un conditionnement myéloablatif pour éviter le rejet de greffe et pour créer un espace à l'intérieur du microenvironnement de la moelle hôte. Ainsi, une transplantation de moelle osseuse requiert une myéloablation partielle associée à une immunodépression, pour atteindre le chimérisme hématopoïétique mixte nécessaire.

De fortes doses de cellules de la moelle osseuse (MO) peuvent surmonter l'accessibilité limitée des niches disponibles **(9, 10).** Toutefois, même dans le cas de greffes optimales de MO compatibles pour les molécules d'histocompatibilité HLA, des antigènes d'histocompatibilité mineurs (AgMH) peuvent provoquer un rejet immun **(11, 12),** nécessitant une immunodépression soutenue. Des transgènes étrangers exprimés dans les cellules du système hématopoïétique (CSH) constituent des antigènes d'histocompatibilité mineurs, qui peuvent compromettre la prise de greffe de **C**SH.

Parmi les sous-groupes de lymphocytes T régulateurs, les cellules T CD4+CD25+ (Tregs), gouvernées par le facteur de transcription forkhead Foxp3, sont particulièrement prometteuses pour l'induction de tolérance en transplantation **(12, 13)** puisqu'elles peuvent prévenir la maladie du greffon contre l'hôte **(14, 15)** et inhiber le rejet d'allogreffe de MO chez des receveurs irradiés de manière sublétale **(16).** Bien que la reconnaissance d'antigènes **(17)** soit suspectée d'être impliquée dans la régulation immune par les Tregs, leur mode d'action *in vivo* reste à élucider. Les modalités de l'emploi de cette population cellulaire pour les applications thérapeutiques potentielles sont en outre encore à définir.

Le problème technique que se propose de résoudre la présente invention est donc de trouver un traitement alternatif au traitement immunodépressif myéloabalatif traditionnel, réputé long, lourd et potentiellement dangereux vis-à-vis de risques infectieux et tumoraux qu'il génère généralement chez le receveur.

Dans le cadre de la présente invention, le Demandeur a mis en évidence le potentiel immunodépressif antigène-spécifique d'une classe particulière de cellules T régulatrices, qui constitue une alternative pour le conditionnement du receveur en vue par exemple d'une transplantation. Ainsi, un conditionnement simple avec cette population cellulaire permet d'augmenter à long terme la tolérance immunitaire d'un hôte histocompatible.

Selon un premier aspect, l'invention concerne donc une composition pharmaceutique comprenant :
- des cellules T régulatrices CD4+ CD25+ spécifiques d'au moins un antigène mineur d'histocompatibilité; et
- des cellules souches, avantageusement hématopoïétiques, porteuses d'au moins ledit antigène.

Tout d'abord, il est à préciser que dans la suite de la description, l'invention concerne l'introduction de cellules ou de tissus dans des hôtes histocompatibles. Ceci implique que le greffon et l'hôte possèdent un système d'histocompatibilité majeur (HLA) identique et sont donc haploidentiques. On parle alors d'une homogreffe.

Dans ce cas, la réaction voire le rejet de la greffe par l'hôte est liée à la présence, dans les CSH greffées, d'antigènes non HLA, dits antigènes mineurs d'histocompatibilité (AgMH), spécifiques de la greffe (et donc du donneur) et divergents voire absents chez l'hôte (receveur). Ces antigènes mineurs, provenant aussi bien de protéines intracellulaires que de protéines de surface, sont présentés dans le contexte de molécules du système HLA.

A titre d'illustration, le transfert de cellules ou de tissus mâles dans un hôte femelle pourtant histocompatible est susceptible de déclencher une réaction immunitaire, en raison de la présence d'antigènes mineurs mâles sur les cellules ou le tissu. Ces antigènes mâles sont codés par le chromosome Y et sont préférentiellement les protéines DBY, UTY ou SMCY. Ces antigènes particuliers présentent l'avantage d'être correctement exprimés (efficaces) et bien conservés (peu de polymorphisme) chez les différents individus, ce qui en fait des antigènes dits universels.

Le principe actif d'une composition pharmaceutique selon l'invention réside dans des cellules T régulatrices CD4+ CD25+ spécifiques d'au moins un antigène AgMH tel que défini ci-dessus. En d'autres termes, ces cellules T régulatrices sont capables de reconnaître spécifiquement cet AgMH par le truchement de leur récepteur T ou de tout autre mécanisme de reconnaissance spécifique.

Les cellules T régulatrices (Treg) correspondent à une sous-population des lymphocytes T -cellules du sang impliquées dans le bon fonctionnement du système immunitaire-, capables de neutraliser en périphérie l'action destructrice des lymphocytes T autoréactifs. Ces cellules régulatrices sont impliquées dans la prévention du déclenchement de réactions immunes auto-réactives, à la base des pathologies auto-immunes. Les cellules T régulatrices CD4+ CD25+ sont aisément identifiées, isolées et purifiées grâce à la présence des marqueurs CD4 et CD2. Elles se caractérisent également par le fait qu'elles expriment spécifiquement le facteur de transcription Foxp3, récemment mis en évidence dans cette population cellulaire.

On entend par "spécifiques d'un antigène" le fait que les cellules T expriment des récepteurs de surface TCR aptes à reconnaître et à interagir avec au moins un épitope de l'antigène AgMH. De manière équivalente, on dit que les cellules T sont dirigées contre lesdits antigènes.

Ces cellules Treg sont aptes à reconnaître au moins un antigène. Cela signifie qu'elles peuvent être monospécifiques, reconnaissant un épitope d'un antigène. Alternativement, elles peuvent être dirigées contre plusieurs antigènes AgMH du donneur. Il peut également être envisagé de diriger ces cellules contre plusieurs épitopes distincts d'un même antigène.

Avantageusement, les cellules T mises en oeuvre dans le cadre de l'invention sont spécifiques d'un unique antigène AgMH, ce qui prouve la puissance de la solution technique proposée, capable de conférer une large tolérance immunitaire.

Dans le cas particulier de la reconnaissance de DBY, une telle population cellulaire a été produite par des souris portant un récepteur aux cellules T transgénique, spécifique du peptide DBY (NAGFNSNRANSSRSS) (SEQ ID 1), complexé avec IA^{b}. De telles souris sont connues sous le nom de souris Marylin (**18**).

De telles cellules T, mono-spécifiques, peuvent maintenant être cultivées et multipliées *in vitro,* grâce au protocole décrit par Tarbel *et al.* (**19**). En outre, il est également possible de générer ce type cellulaire *de novo* chez l'homme (**20**).

A terme, une composition pharmaceutique selon l'invention contiendra les facteurs nécessaires à la génération *de novo* chez l'hôte de cellules T telles que définies ci-dessus.

Dans un mode de réalisation avantageux, les cellules T telles que définies ci-dessus sont autologues, c'est à dire qu'elles sont issues du donneur qui, dans ce cas particulier, est également le receveur.

En combinaison avec cette première population cellulaire (Tregs), la composition pharmaceutique comprend en outre un second type de cellules, à savoir des cellules souches. Pour des raisons éthiques, ne sont pas concernées par la présente invention les cellules souches directement issues d'embryons humains.

Ces cellules sont porteuses et expriment au moins l'antigène qui diffère chez le receveur, mais qui est apte à être reconnu par les cellules Treg de la composition pharmaceutique selon l'invention.

Dans un premier mode de réalisation, ces cellules sont issues du donneur histocompatible et portent « naturellement » (codé dans leur patrimoine génétique) cet antigène, absent chez le receveur.

Alternativement, il peut s'agir de cellules autologues issues du receveur (qui constitue donc également le donneur), dans lesquelles au moins un antigène AgMH a été introduit. Préférentiellement, ceci est réalisé par transfert de gènes à l'aide d'outils connus de l'homme du métier, notamment des vecteurs viraux.

En pratique, il s'avère que l'hôte receveur d'une telle composition va acquérir une tolérance immune envers l'antigène reconnu spécifiquement par les Tregs selon l'invention, et va de ce fait développer également un état de tolérance envers l'ensemble des antigènes portés par ces cellules souches, initialement reconnus par les cellules T régulatrices spécifiques.

Dans le cas particulier du transfert mâle/femelle, il peut s'agir de cellules issues d'un donneur mâle et donc porteuses des antigènes mineurs mâles tels que DBY, UTY et SMCY, par exemple des cellules mâles de la moelle osseuse (MO).

Alternativement, il peut s'agir de cellules de la moelle osseuse issues du receveur femelle, transduites par des vecteurs porteurs des gènes codant DBY et/ou UTY et/ou SMCY. L'utilisation de cellules autologues présente un avantage évident en termes de compatibilité.

De manière générale et avantageuse, il s'agit de cellules souches hématopoïétiques (CSH) qui assureront le maintien à long terme de l'état de tolérance, sans conditionnement myéloablatif ni immunosuppresseur chez le receveur. Ces CSH d'origine médullaire ou dérivées de diverses populations de cellules progénitrices non-embryonnaires pourront aussi posséder une pouvoir thérapeutique direct soit par leur vertu de maintien de l'état de tolérance spécifique vis-à-vis d'un ou plusieurs AgMH, soit en permettant la production de facteur métaboliques permettant la correction de désordres d'origine génétique.

Dans un premier mode de réalisation, il s'agit de cellules de la moelle osseuse (MO) contenant des cellules souches hématopoïétiques (CSH). Ces cellules pluripotentes sont destinées à donner naissance aux différents types de cellules sanguines au cours de l'hématopoïèse. Ces cellules sont classiquement extraites de prélèvement médullaires ou sanguins et purifiées selon des marqueurs phénotypiques (par exemple CD34), ou enfin enrichies selon des critères fonctionnels, tels que leur capacité d'exclusion du marqueur Hoechst. Dans ce second mode de réalisation, les cellules souches sont également de nature hématopoïétique, mais subissent une purification initiale afin d'isoler une fraction enrichie en cellules progénitrices. Cette méthode élimine un grand nombre de cellules qui ne possèdent pas le pouvoir de s'implanter dans le receveur et permet la modification génétique efficace d'un nombre limité de cellules.

Dans le cadre de l'invention, il est également envisagé d'utiliser des cellules souches modifiées génétiquement, porteuses d'un transgène d'intérêt. Ces transgènes peuvent être apportés à l'aide d'un vecteur, préférentiellement viral. Les vecteurs lentiviraux sont préférés dans le cadre des applications thérapeutiques impliquant des CSH. Le produit de ces transgènes est également perçu comme un antigène mineur d'histocompatibilité de la part de l'hôte receveur d'une composition pharmaceutique selon l'invention. Ce transgène peut donc être exprimé en plus de l'antigène spécifiquement reconnu par les cellules Treg selon l'invention ou bien peut lui-même constituer cet antigène.

De manière remarquable, la composition selon l'invention permet d'assurer l'expression stable du transgène.

A titre de transgène d'intérêt, on peut par exemple envisager tout gène fonctionnel apte à compenser un gène défectueux chez l'hôte, par exemple le gène de la dystrophine chez les patients atteints de DMD.

La composition visée par l'invention peut contenir en outre des adjuvants ou des véhicules pharmaceutiquement acceptables, connus de l'homme du métier.

La quantité de cellules dans la composition pharmaceutique revendiquée est avantageusement administrée à hauteur de 10⁴ et 10⁸ cellules par kg du receveur. Dans le cadre de l'invention, il a été montré qu'une quantité même faible des cellules Treg suffisait à un conditionnement efficace. La quantité en cellules Treg est avantageusement égale à environ 10⁶ cellules par kg.

L'injection par voie sanguine, préférentiellement en intraveineuse, est le mode d'administration privilégié de la composition pharmaceutique selon l'invention. Il a été constaté qu'une injection unique était aussi efficace qu'une succession d'injections répétées (hebdomadaires) de quantités équivalentes de cellules.

La présence des deux types cellulaires dans la composition pharmaceutique revendiquée permet de les introduire simultanément, au cours d'une injection unique, dans l'hôte receveur histocompatible. Toutefois, l'effet avantageux sur la tolérance immunitaire (utile en thérapie génique et cellulaire, pour les transplantations et les greffes) d'une telle combinaison est conservé aussi bien lors d'une administration simultanée, séparée ou étalée dans le temps des 2 types cellulaires décrits ci-dessus, faisant l'objet de la composition selon l'invention.

Un autre aspect de l'invention concerne l'utilisation d'une telle composition pour la préparation d'un médicament destiné à augmenter ou améliorer la tolérance immunitaire chez un hôte histocompatible.

Pour rappel, un hôte histocompatible est le receveur d'un greffon issu d'un donneur possédant des antigènes majeurs compatibles mais des antigènes mineurs divergents, susceptibles de déclencher une réaction immunitaire aboutissant au rejet du greffon et/ou du transgène thérapeutique par l'hôte.

Dans le cadre de la présente invention, plusieurs observations de première importance pour les perspectives thérapeutiques ont été réalisées après conditionnement avec les deux types cellulaires tels que décrits :
- la tolérance immunitaire du receveur est améliorée à court terme comme à long terme ;
- la tolérance reste spécifique puisque le receveur reste parfaitement immunocompétent ;
- la tolérance s'établit vis-à-vis de l'antigène mineur spécifique reconnu par les cellules Treg, mais également vis-à-vis d'autres antigènes du même type, y compris vis-à-vis d'un transgène associé initialement dans le greffon dont l'expression stable est assurée ;
- la tolérance persiste vis-à-vis d'une greffe secondaire portant des antigènes mineurs, même si l'antigène initial spécifiquement reconnu par les cellules Treg n'est pas présent dans la greffe secondaire.

En pratique, ces avantages offrent différentes applications thérapeutiques concernant les greffes de tissus autologues génétiquement modifiés, les procédures de transfert de gènes et enfin des procédures d'induction de tolérance spécifiques d'antigènes définis visant à atténuer les réactions auto-réactives dans des pathologies auto-immunes possédant des cibles moléculaires et cellulaires identifiées.

Tout d'abord, le fait de pouvoir augmenter la tolérance immunitaire du receveur permet une plus grande flexibilité au niveau du choix du donneur, en particulier en abolissant la barrière de la différence de sexe.

Dans le cas de greffage de tissus liquides ou solides, par exemple celui de la peau, le greffage des cellules souches n'est qu'une étape préliminaire de conditionnement, préparatoire à la greffe secondaire du tissu d'intérêt, issu du donneur.

L'invention concerne donc également l'utilisation de la composition décrite dans le cas où la tolérance immunitaire est augmentée en vue de la transplantation d'un tissu issu d'un donneur porteur d'un antigène mineur d'histocompatibilité, par exemple pour une greffe de peau.

Les cellules souches peuvent être le vecteur d'un ou plusieurs gènes dits réparateurs servant à corriger un défaut d'origine génétique présent chez l'hôte. Le gène réparateur peut être apporté par le patrimoine génétique des cellules souches elles-mêmes ou introduit dans ces cellules au moyen d'un vecteur, préférentiellement viral. Il s'agit dans ce dernier cas d'un transgène porté par les cellules souches.

Comme précédemment décrit, le conditionnement décrit satisfait aux applications thérapeutiques revendiquées. Le médicament selon l'invention constitue donc un traitement suffisant en tant que tel.

L'invention et les avantages qui en découlent ressortiront mieux des exemples de réalisation suivants à l'appui des figures annexées, qui ne sont en aucun cas limitatifs.

Etant donné que la prise de greffe de tissus mâles chez des receveuses femelles génère une réponse immune bien définie contre les antigènes DBY, UTY et SMCY dans des souris C57B1/6 (B6) (**11**), l'exemple de réalisation suivant est basé sur ce modèle pour tester si les Tregs mâle-spécifiques peuvent promouvoir la prise de greffe à long terme de cellules progénitrices de la moelle épinière. Ce modèle mime les situations dans lequelles des cellules souches autologues, transduites avec des transgènes étrangers, sont rejetées en raison des réponses spécifiques au transgène de l'hôte. De plus, la présence de plusieurs antigènes mâles introduit un niveau de complexité appréciable, permettant de tester dans qùelle mesure les Tregs dirigées contre un épitope unique peuvent supprimer les réponses immunes induites contre toutes les autres protéines exprimées. Des Tregs dirigées contre l'antigène mâle DBY, obtenues à partir de souris Marylin porteuses d'un TCR transgénique a été utilisé comme seul conditionnement pour obtenir une prise de greffe à long terme de doses appréciables de MO mâles chez des hôtes femelles B6. De manière importante pour les applications thérapeutiques, les souris B6 rendues tolérantes par cette procédure deviennent permissives envers des prises de greffe secondaires de tissus autologues modifiés avec le transgène présent dans les MO initiales, indépendamment de l'antigène DBY reconnu par les Tregs perfusées.

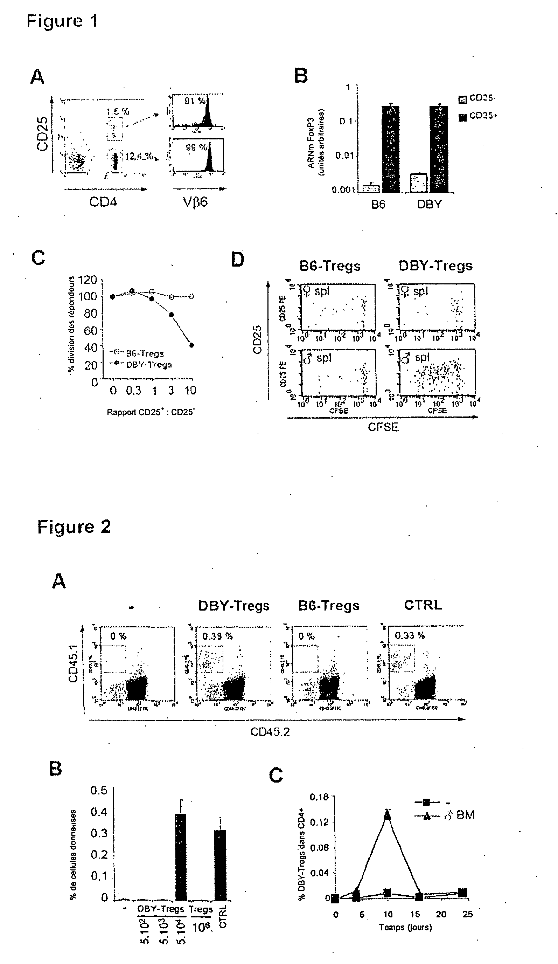

### Figure 1: Expansion in vivo et propriétés immunosuppressives des Tregs-DBY.

(**A**) Analyse par FACS des cellules des ganglions lymphatiques d'une souris femelle Marylin marquées avec anti-CD4-FITC, Vβ6-PE et CD25-biotine/APC-streptavidine. Les cellules mortes ont été exclues à l'aide d'actinomycine D (7-AAD).
(**B**) Les niveaux d'ARNm FoxP3 des cellules CD25+ et CD25- purifiées à partir de souris femelles B6 et Marylin ont été déterminés par analyse PCR en temps réel sur des splénocytes frais.
(**C**) Suppression *in vitro* par les Tregs-DBY, estimée par le pourcentage de division de 5.10³ cellules T-helper CD4+CD25- d'une souris Marylin (marquées avec 2µM de CFSE), stimulée avec 5.10⁵ splénocytes mâles de B6 en présence de doses variables de Tregs-DBY (cercles pleins) ou de Tregs-B6 non-spécifiques (cercles vides).
(**D**) Expansion *in vivo* de Tregs-DBY: des Tregs-DBY marquées avec 5 µM de CFSE ont été transférées conjointement avec 10.10⁶ splénocytes femelles ou mâles dans une souris femelle receveuse CD45.1. Au jour 6, les splénocytes ont été marqués avec CD4-Cy, CD25-PE et CD45.2-biotine/APC-streptavidine. Les histogrammes de points sont fenêtrés sur les cellules CD4+CD45.2+.

### Figure 2: Prise de greffe à court terme de MO mâle et expansion transitoire des Tregs-DBY.

(**A, B**) Prise de greffe à court terme (Jour 28) de cellules de MO mâle congéniques CD45.1 (5.10⁶ cellules), transférées dans des souris femelles B6, soit non traitées (-) ou conditionnées avec une injection intraveineuse de quantités variables de Tregs-DBY ou 1.10⁶ B6-Tregs. Des souris mâles B6 ont été greffées, comme contrôle positif (CTRL). Les pourcentages de cellules donneuses CD45.1+ ont été analysés dans les PBMC. Les résultats représentent la moyenne de 3-6 souris par groupe ± l'erreur standard de la moyenne (SEM).
(C) Expression transitoire de Tregs-DBY. Tregs-DBY (1.10⁵ cellules) ont été transférées chez une souris femelle B6 avec ou sans MO mâle (10⁷ cellules). A chaque temps, 2 souris ont été sacrifiées et leurs splénocytes colorés avec CD45.1-PE, APC-CD4 et 7-AAD. Le graphe représente le pourcentage de cellules CD45.1+ dans les cellules CD4+ 7-AAD-. Les résultats représentent la moyenne de 2 souris par groupe ± SEM.

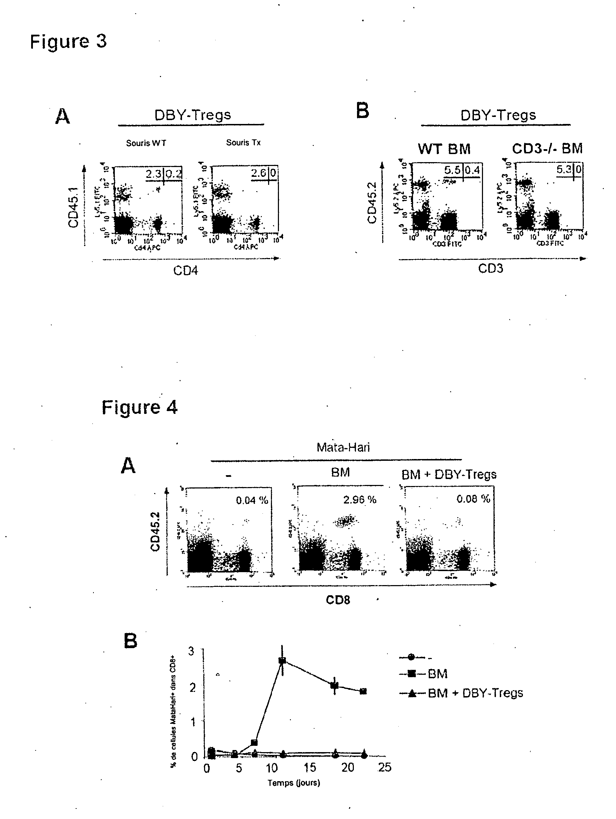

### Figure 3: La tolérance envers les antigènes mâles a lieu principalement via des mécanismes périphériques.

(A) Des MO mâle de souris femelles congéniques CD45.1 B6 (15.10⁶ cellules) ont été transférées dans des souris B6 intactes (n=5) ou thymectomisées (n=5), conditionnées avec une injection intraveineuse unique de 1.10⁵ Tregs-DBY.
(B) Des MO mâle de souris sauvages B6 ou de souris CD3 -/- (15.10⁶ cellules) ont été transférées dans des souris congéniques B6 femelles CD45.1 (n=5 pour chaque groupe), conditionnées avec une injection intraveineuse unique de 1.10⁵ Tregs-DBY. Dans (A) et (B), le chimérisme donneur exprimé en % de cellules CD45.1+ a été analysé dans les PBMC à différents temps après transfert de MO. Les colorations FACS représentées (Jour 60) sont représentatives de deux expériences.

### Figure 4: Le chimérisme mixte mâle-femelle altère les cellules T spécifiques des antigènes mâles.

10⁵ cellules T Mata-Hari CD8 ont été transférées dans des souris femelles congéniques CD45.1 soit seules (-), soit avec 10.10⁶ MO mâles ou avec 10.10⁶ MO mâles et 10⁵ Tregs-DBY.
(**A**) Des échantillons sanguins représentatifs colorés avec CD45.2-biotine/APC-streptavidine, CD8-PE et 7-AAD sont montrés (Jour 11 après transfert).
(**B**) Le pourcentage de cellules Mata-Hari parmi les CD8+ totales analysées dans les PBMC à différents temps est montré. Les résultats représentent la moyenne de 2 souris par groupe ± SEM.

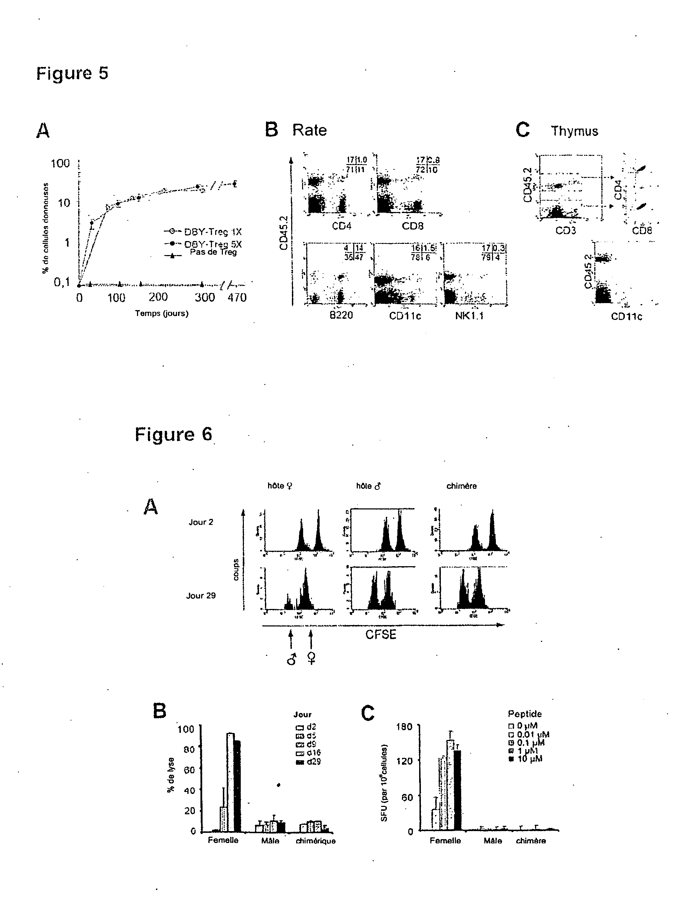

### Figure 5: Développement d'un chimérisme mixte à long terme et multi-lignage.

(A) Des cellules MO mâles B6 (15.10⁶ cellules) ont été transférées dans des souris femelles B6 CD45.1 congéniques, conditionnées avec soit 5 injections intraveineuses hebdomadaires de 2-5.10⁵ Tregs-DBY (cercles pleins), ou une injection unique de 1.10⁵ Tregs-DBY (cercles ouverts). Le chimérisme donneur exprimé en % de cellules CD45.2+ a été analysé dans les PBMC à différents temps après transfert de MO. Les résultats représentent la moyenne de 5 souris par groupe ± SEM.
(**B, C**) Des souris chimérisées depuis plus de 300 jours (5 injections de Tregs-DBY) ont été sacrifiées et les cellules de différents organes ont été analysées par FACS. Les splénocytes ont été colorés avec CD45.2-biotine/APC-streptavidine, **PE** conjugué avec anti-CD8, CD4, B220, CD11c et 7-AAD (**B**). Des thymocytes (C) ont été colorés avec CD4-FITC, CD3-PE, CD45.2-biotine/PECy7-streptavidine et CD8-APC (pas de fenêtre) ou avec CD3-FITC, CD45.2-biotine/PE-streptavidine, 7-AAD et CD11c-APC (FACS fenêtré sur CD3-7-AAD est montré).

### Figure 6: Forte tolérance vis-à-vis des antigènes mâles dans des souris chimériques.

(A, B) Absence d'activité CTL anti-mâle dans les souris chimériques. Les splénocytes mâles et femelles marqués avec 0,5 µM et 5 µM de CFSE, respectivement, ont été transférés dans des souris femelles, des souris mâles ou des souris femelles chimériques. Les PBMC ont été marquées avec du PE anti-B220 et 7-AAD à différents temps et les pourcentages de lyse spécifique des splénocytes mâles sur femelles ont été calculés, comme détaillé dans la partie Matériel et Méthodes. Les résultats représentent la moyenne de 2 souris par groupe ± SEM.
(**C**) Absence de réponse anti-mâle par les cellules T. Des souris femelles, mâles ou chimériques ont été provoquées de manière sub-cutanée avec 50 µg de peptide UTY émulsifié dans IFA. Les splénocytes ont été testés au jour 10 dans un test standard IFNγ ELISPOT contre différentes doses de peptide UTY. Les résultats représentent la moyenne de 3 souris par groupe ± SEM.

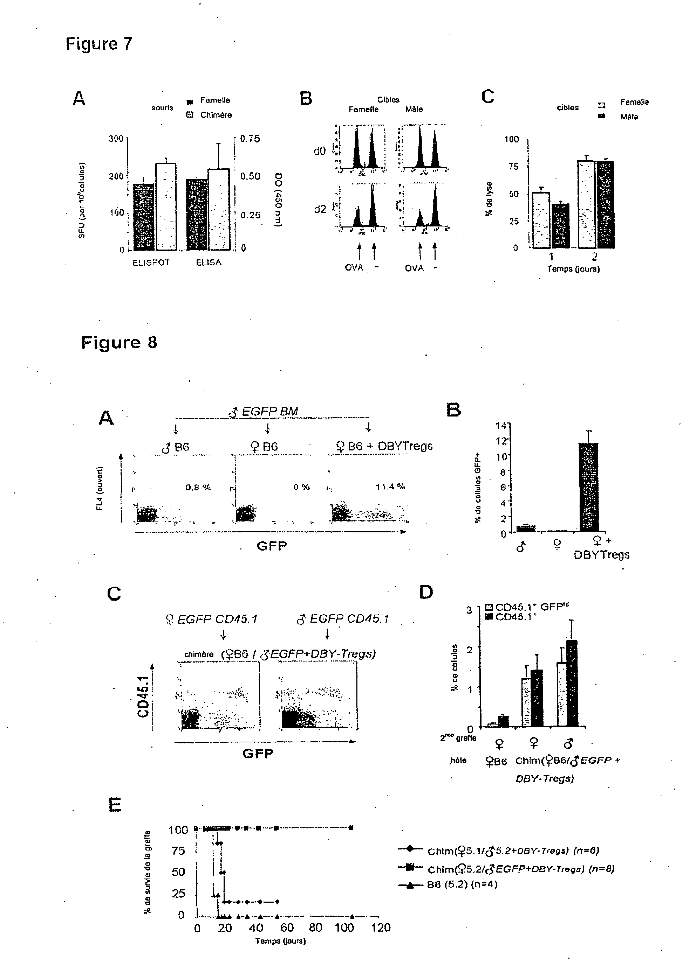

### Figure 7: Le chimérisme hématopoïétique n'affecte pas la réponse immune envers des antigènes tiers.

(**A**) Des souris femelles chimériques ou naïves ont été provoquées de manière sub-cutanée avec 100 µg de protéine OVA émulsifiée dans IFA. Les splénocytes ont été testés au jour 10 dans un test standard IFNγ ELISPOT contre l'épitope OVA257. Les sérums ont été testés par **ELISA** pour les anticorps anti-OVA. Les résultats représentent la moyenne de 3 souris par groupe ± SEM.
(**B, C**) Susceptibilité des cellules mâles à l'activité immune cytolytique. Des souris chimériques ont été provoquées de manière sub-cutanée avec de la protéine OVA dans IFA comme dans (A), et ont été perfusées au jour 8 avec des splénocytes mâles (barres noires) ou des splénocytes femelles (barres grises), pulsés avec OVA257 (0,5 µM de CFSE) ou non pulsés (5 µM de CFSE). Les PBMC ont été analysées aux jours 0, 1 et 2. Les résultats représentent la moyenne de 2 souris par groupe ± SEM. Le pourcentage de lyse spécifiques des cellules pulsées sur les non pulsées a été calculé comme précédemment (Fig. 6) avec les cellules mâles sur femelles.

### Figure 8: Prise de greffe secondaire de tissus exprimant le transgène EGFP en absence de l'antigène DBY.

(A) 7.10⁶ cellules MO mâles de souris transgéniques EGFP ont été transférées dans des hôtes mâles B6, dans des hôtes femelles B6, ou dans des hôtes femelles B6 conditionnées avec 10⁵ Tregs-DBY. L'analyse FACS représentative de 3 expériences est montrée (Jour 150).
(**B**) Chimérisme donneur exprimé en % des cellules EGFP+ analysé dans les PBMC 5 mois après le transfert de MO EGFP selon (A). Les résultats représentent la moyenne de 3 souris par roupe ± SEM.
(**C, D**) Des cellules MO issues de souris mâles ou femelles EGFPxCD45.1 ont été transférées dans des souris femelles EGFP chimériques ou naïves. Les relevés représentatifs de FACS (C) et le pourcentage des cellules EGFP^{high} et CD45.1⁺ (**D**) dans les PBMC à 5 mois sont montrés. Les résultats représentent la moyenne de 3 souris par groupe ± SEM.
(**E**) Prise de greffe à long terme de greffes de peau femelle EGFP dans des souris chimériques mâle-femelle EGFP (n=8, carrés pleins). Dans les contrôles, 5/6 des souris chimériques CD45.2/CD45.1 dépourvues d'EGFP (carreaux pleins) et 4/4 des souris B6 femelles (triangles pleins) ont rejeté la greffe de peau femelle EGFP entre le jour 12 et le jour 16. Les résultats présentés sont issus de deux expériences indépendantes.

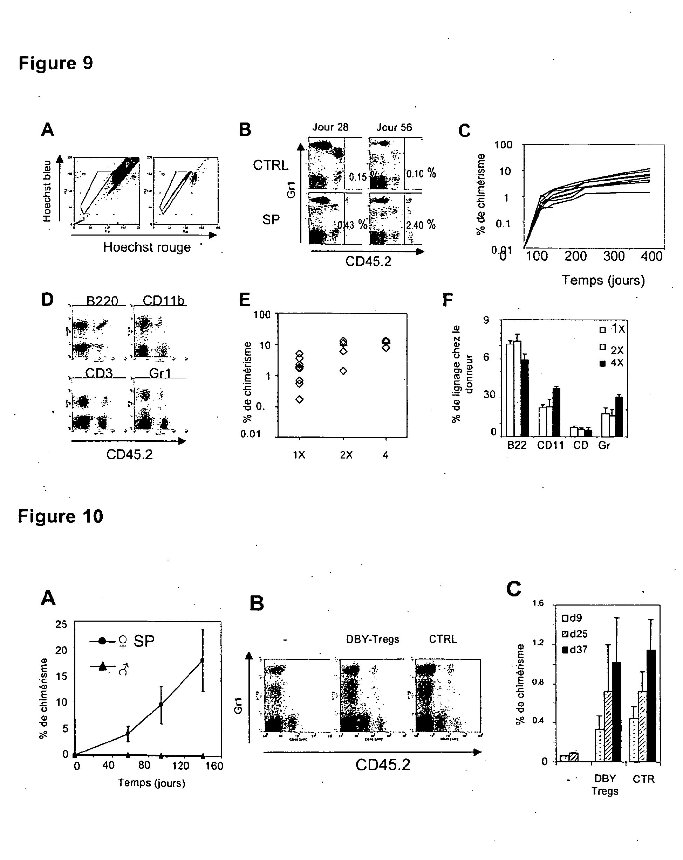

### Figure 9 : Greffe de cellules souches hématopoïétiques purifiées sans pré-conditionnement de l'hôte

A) Analyse par FACS du profile SP de cellules de moelle osseuse, en absence ou en présence de verapamil. B-D) Etablissement d'un chimérisme hématopoïétique au cours du temps. Des souris femelles receveuses CD45.1 ont été injectées par voie intraveineuse 4 jours de suite avec du PBS (CTRL) ou avec 10⁵ cellules SP provenant de souris B6 (SP). Le pourcentage de cellules donneuses a été analysé dans les PBMC par FACS. Données représentatives à jour 28 et jour 56 (B); suivi au cours du temps souris par souris, groupe SP (C) et greffe multi-lignage, groupe SP (D). E-F) Des souris femelles receveuses CD45.1 ont été injectées par voie intraveineuse avec 105 cellules SP provenant de souris B6 4 jours de suite (4X), 2 jours de suite (2X) ou seulement à J0 (1X). Pourcentage de chimérisme dans les PBMC (E) et reconstitution multi-lignage (F) sont indiqués. Les résultats représentent la moyenne de 3-8 souris par groupe ± l'erreur standard de la moyenne (SEM).

### Fig. 10 : Des cellules souches hématopoïétiques non appariées sur des antigènes mineurs peuvent greffer en présence de Tregs spécifiques

A) Rejet de CSH mâles par des souris femelles. Des souris femelles receveuses CD45.1 ont été injectées par voie intraveineuse à J0 avec 10⁵ cellules SP provenant de souris B6 femelles ou mâles. Le pourcentage de cellules donneuses a été analysé dans les PBMC par FACS au cours du temps. Les résultats représentent la moyenne de 3 souris par groupe ± l'erreur standard de la moyenne (SEM). B-C) Le rejet est inhibé par des Tregs. Des souris femelles receveuses CD45.1 ont été injectées par voie intraveineuse à J0 avec 10⁵ cellules SP provenant de souris B6 mâles en absence (NO) ou en présence de 3.10⁵ Tregs-DBY (DBY-Tregs). Dans le groupe contrôle (CTRL), des souris receveuses CD45.1 mâles ont été injectées avec des SP provenant de souris B6 mâles. Le pourcentage de cellules donneuses a été analysé dans les PBMC par FACS au cours du temps. Les résultats représentent la moyenne de 3 souris par groupe ± l'erreur standard de la moyenne (SEM).

### MATERIEL ET METHODES

### Souris

Des souris âgées de 6 à 8 semaines C57BI/6 (CD45.2) et congéniques Ly5.1 (PtprcaPep3b/BoyJ [CD45.1]) ont été obtenues auprès de Charles River (L'Arbresle, France). Des souris Marilyn portant un récepteur aux cellules T transgénique, spécifique du peptide DBY (NAGFNSNRANSSRSS) (SEQ ID 1), complexé avec IA^{b} (**18**) ont été données par O. Lantz et les femelles ont été utilisées ici sur un fond génétique B6 RAG2+/- (CDTA, Orléans, France). Des souris Mata-Hari RAG1 -/portant un TCR transgénique spécifique du peptide UTY (WMHHNMDLI) (SEQ ID 2) complexé avec D^{b} (**21**) ont également été données par O. Lantz. Des souris hémizygotes transgéniques EGFP (C57BL/6-Tg(ACTB-EGFP) 1Osb/J, Jackson Laboratory, USA) expriment l'ADNc de EGFP sous le contrôle d'un promoteur de la béta-actine du poulet et l'amplificateur du cytomégalovirus. Des souris EGFP ont également été croisées avec des souris CD45.1 pour générer des souris EGFP x CD45.1. Les deux espèces ont été élevées dans nos installations animalières. Toutes les expériences animales ont été réalisées selon les directives institutionnelles pour le soin et l'usage des animaux.

### Analyse par FACS (Fluorescence-activated cell sorting)

Tous les réactifs étaient de BD-PharMingen (Le Pont de Claix, France). Les globules rouges ont été éliminés par choc hypotonique avec du tampon PharMLysis. Les cellules mononucléaires du sang périphérique (PBMC) ont été incubées pendant 10 minutes à 4 °C avec des anticorps 2.4G2 contre les récepteurs FcII/III, puis colorées 30 minutes dans du PBS - 0,1% sérum-albumine bovin avec des quantités saturantes de combinaisons des mABs suivants : anti-CD3, anti-CD4, anti-CD11c, anti-B220 et anti-CD45.1 conjugués à la fluorescéine isothiocyanate (FITC), anti-CD8, anti-CD11b, anti-CD25, anti-Gr1, anti-CD45.2, anti-NK1.1 et anti-Vβ6 conjugués la phycoérythrine (PE), anti-CD45.2 conjugués à la biotine et streptavidine conjuguée à l'allophycocyanine (APC). Les cellules mortes ont été exclues en utilisant une coloration à la 7-actinomycine D (Sigma Chemical Co, St., MO). L'analyse par cytométrie en flux a été réalisée sur un FACSCalibur, en utilisant le logiciel CELLQuest (BD).

### Purification des cellules T régulatrices

Les splénocytes et les cellules des ganglions lymphatiques ont été incubés avec des quantités saturantes d'anti-CD25 biotinylés (7D4) et des microbilles de streptavidine (Miltenyi Biotec, Paris, France), suivi par une séparation magnétique des cellules en utilisant des colonnes LS (Miltenyi Biotec), conformément aux instructions du fabricant. Les cellules ont ensuite été colorées 30 minutes sur la glace avec streptavidine-FITC, triées sur un MoFlow (DakoCytomation, Freiburg, Germany) et injectées dans la veine de la queue, dans 0,2 ml de PBS.

### Analyse de l'inhibition in vitro

Des cellules T CD45.2 CD4+CD25- purifiées, issues de souris Marylin, ont été marquées avec CFSE (Molecular Probes, Cambridge, United Kingdom). Brièvement, 2x10⁷ cellules/mL ont été incubées avec 2 µM de CFSE à 37° C pendant 10 minutes dans RPMI 1640, avant d'être lavées 2 fois. Ensuite 5.10³ cellules ont été cultivées dans des plaques à fond en U avec 5.10⁵ splénocytes mâles de souris congéniques CD45.1 et différentes proportions de Tregs CD4+CD25+ issues de souris B6 ou Marylin. Au jour 3, les cellules ont été colorées avec CD4-PE, 7-AAD et CD45.2-biotine/streptavidine-APC avant l'analyse par FACS. La division (en pourcent) des cellules CD25- répondant a été déterminée comme le pourcentage des cellules ayant subi au moins deux divisions par rapport à la quantité totale des cellules répondant qui s'étaient divisées en absence de Tregs.

### Transplantation de moelle osseuse

La moelle osseuse donneuse a été extraite avec du PBS des fémurs et des tibias. Les globules rouges ont été lysés avec du tampon ACK et les cellules de la moelle osseuse (BM) ont été injectées dans la veine de la queue dans 0,2 mL de PBS 1 x en présence ou en absence de Tregs CD4+CD25+.

### Analyse de la mortalité in vivo

Des cellules spléniques C57BI/6 femelles et mâles, ou des cellules spléniques sexe-assorties porteuses ou non de 10 µM OVA257 (2 x 10⁷ /mL dans RPMI 1640) ont été incubées avec 5 µM ou 0,5 µM CFSE (Molecular Probes, Cambridge, United Kingdom) à 37° C pendant 10 min. Après lavage, les cellules ont été mélangées, et 2 x 10⁷ cellules ont été injectées dans la veine de la queue dans 0,2 mL PBS 1x. Les PBMC ont été collectées à partir de souris individuelles à des intervalles de temps réguliers, marquées avec PE anti-B220 et 7-AAD et analysées pour l'expression CFSE par FACS. Le pourcentage de lyse spécifique des cellules mâles par rapport aux femelles (m/f) a été calculé sur les cellules B220+ à chaque temps tₓ comme suit : % de lyse spécifique = [(m/f)t₀ - (m/f)tₓ] / [(m/f)t₀] x 100. Les quantités m et f sont mesurées sur des portes standards placées sur les pics des histogrammes CFSE mâle bas et femelle haut.

### Réponses immunes anti-ovalbumine

Les souris ont subi une provocation sous-cutanée à la base de la queue avec 100 µg de protéine ovalbumine (Sigma) ou 50 µg du peptide OVA257 (SIINFEKL) (Epytop, Nîmes, France), émulsifiés dans un adjuvant de Freund incomplet (Difco laboratories, BD). Des analyses par IFNγ-ELISPOT et ELISA ont été réalisées 8 à 10 jours plus tard, comme déjà décrit (17). Brièvement, pour l'analyse en IFNγ-ELISPOT, des splénocytes fraîchement isolés (2x10⁶/puits et des dilutions en série) ont été cultivés en milieu complet avec 10 µM de OVA257 ou sans. Pour chaque essai, Con A a été ajouté (5µg/ml) comme contrôle positif. Après 20 h, les spots ont été révélés et comptés en utilisant un Bioreader 2000 (BIO-SYS, Karben, Germany). Les unités de formation des spots (SFU) sont représentées après soustraction des valeurs de bruit de fond obtenues avec des splénocytes non pulsés.

### Analyse par PCR en temps réel

Les ARN totaux ont été extraits en utilisant le kit RNAeasy Micro Kit (QIAGEN) à partir de 10⁵ cellules isolées de chaque population. L'ADNc a été synthétisé à partir de chaque lot d'ARN en utilisant la transcriptase inverse MuLV et des hexamères aléatoires comme amorces (Applied Biosystems). La PCR en temps réel a été réalisée sur un ABI prism 7700 en utilisant Absolute QPCR ROX Mix (ABgene) en double et les cycles moyens de seuil (Ct) des doubles ont été utilisés pour calculer le niveau d'ARNm Foxp3 dans chaque population. Tous les niveaux rapportés d'ARNm ont été normalisés par rapport au niveau de l'ARMm mPO, avec mPO=1. Les amorces de PCR ont été comme suit :
- Foxp3:
   5'-GGCCCTTCTCCAGGACAGA-3' (SEQ ID 3)
   5'-GCTGATCATGGCTGGGTTGT-3' (SEQ ID 4)
   5'-ACTTCATGCATCAGTCTCCACTGTGGAT-3' (SEQ ID 5)
- mPO:
   5'-CTCCAAGCAGATGCAGCAGA-3' (SEQ ID 6)
   5'-ATAGCCTTGCGCATCATGGT-3' (SEQ ID 7)
   5'CCGTGGTGCTGATGGGCAAGAA-3' (SEQ ID 8).

### Greffe de peau

Des greffes de peau de 1 cm de diamètre ont été préparées à partir de dos de souris femelles EGFP et ont été greffées sur les flancs des receveurs, en utilisant un adhésif tissulaire (3M Vetbond, France) à la place d'une couture chirurgicale. Les bandages ont été retirés au jour 5. Les greffes ont été suivies tous les 2 ou 3 jours jusqu'au jour 20, puis chaque semaine, et enregistrées comme rejetées quand moins de 10% de tissu viable persistait.

### Purification des CSH

Les cellules de moelle sont préparées à partir des fémurs et tibias. Après lyse des globules rouges par choc hypotonique, les cellules sont resuspendues à 10⁶/ml dans du PBS supplémenté avec 1% de sérum autologue et marquées avec 6.5 µg/ml de Hoechst 33342 (Sigma) pendant 90 min à 37°C. Les contrôles sont incubés en présence de100µM de verapamil. Les cellules sont alors lavées et marquées avec du 7-actinomycine D (7-AAD, Sigma Chemical Co, St., MO). Le tri et l'analyse des cellules excluant le Hoechst (SP) a été fait sur trieur MoFlow (DakoCytomation, Freiburg, Allemagne). Les cellules SP comptent généralement pour 0.04% à 0.05% des cellules de moelle totales.

### RESULTATS

### Activité antigène-spécifique de cellules Tregs CD4+CD25+ de souris Marylin

Dans la souche B6, les souris femelles rejettent les greffes de MO mâles H2-appariés, générant des réponses des cellules T CD4 contre un peptide DBY dans le contexte de molécules I-A^{b} et des réponses helper CD8 dépendantes contre les épitopes restreints UTY et SMCY (**11**). Comme sources de Treg dirigées contre un antigène mâle donné, des Tregs CD4+CD25+ ont été isolées à partir de souris Marylin exprimant un TCR transgénique, spécifique pour l'épitope mâle restreint I-A^{b} DBY NAGFNSNRANSSRSS (SEQ ID 1) (**18**).

Phénotypiquement, les souris femelles RAG+/- Marylin possède une population distincte de cellules T CD4+CD25+ (DBY-Tregs) exprimant le transgène Vβ6 TCR et représentant 6-11% des cellules T CD4+ totales (Fig. 1A). Le phénotype de ces cellules T régulatrices a été confirmé par la quantification du marqueur de lignage/différentiation pour les Tregs récemment identifié, FoxP3 (Fig. **1B**). Comme détecté par PCR en temps réel, les CD25+ purifiées à partir de souris Marylin expriment des niveaux de FoxP3 similaires à ceux observés dans les T-regs B6 (B6-Tregs) et 80 fois plus élevés que ceux trouvés dans les cellules T CD25-.

La spécificité et la fonction suppressive des DBY-Treg a été caractérisée *in vitro* et l'inhibition de la prolifération des cellules T naïves CD4+CD25- de Marylin (DBY-T-helper), stimulées avec des splénocytes mâles et marquées avec CFSE, a été suivie. Comme montré dans la **Fig. 1C****,** DBY-Tregs inhibent la prolifération des DBY-T-helper de manière dose dépendante, alors que les B6-Tregs non spécifiques sont inefficaces quel que soit le rapport.

Ensuite, la spécificité antigénique des DBY-Tregs a été vérifiée *in vivo* par leur capacité de prolifération dans des receveuses femelles provoquées avec des splénocytes femelles ou mâles. Comme mesuré avec des expériences de dilution. CFSE, une expansion vigoureuse des DBY-Tregs a été observée au jour 6 (Fig. 1D). Cela n'est survenu qu'après immunisation avec des splénocytes mâles. Les B6-Tregs sont restées pour l'essentiel indivisées dans toutes les conditions (Fig. 1D). En conclusion, les DBY-Tregs des souris Marylin se sont avérées efficaces dans les expériences de suppression *in vitro* et ont été activées de manière mâle spécifique *in vivo.*

### Induction du chimérisme hématopoïétique mixte en utilisant les DBY-Tregs

La capacité de ces Tregs mono-spécifiques à induire le chimérisme mixte, sans préconditionnement, a été explorée. Des cellules DBY-Tregs ont été utilisées pour promouvoir la prise de greffe directe d'une dose modérée de MO mâles dans des hôtes femelles B6, en l'absence de myéloablation et d'immunosuppression. Comme contrôle en l'absence de réponses immunes, l'injection de 8.10⁶ MO mâles congéniques CD45.1+ a mené à un chimérisme de 0.3-0.4% dans des souris mâles CD45.2+ (**Fig. 2A**). La même greffe a été complètement rejetée dans des souris femelles CD45.2+ au jour 28. Dans ces souris femelles, la prise de greffe était déjà compromise au jour 14 et pratiquement absente au jour 21 (résultats non montrés). Comme attendu, la perfusion dans des femelles de DBY-Tregs, de manière concomitante avec les MO mâles, a supprimé le rejet de la greffe et a permis d'atteindre près de 0.4% de chimérisme (**Fig. 2A**). La dose de DBY-Tregs a ensuite été modulée et un effet de tout ou rien sur la prise de greffe des MO a été observé entre 5.10⁴ et 5.10³ DBY-Tregs (**Fig. 2B**). De manière importante, les doses de DBY-Tregs au-dessus du seuil ont promu une prise de greffe maximale dans toutes les souris femelles, atteignant un niveau identique à celui dans les receveurs mâles contrôles, traités avec la même quantité de cellules MO. Comme pour la suppression *in vitro,* une dose plus forte de 10⁶ B6-Tregs non spécifiques était inefficace (Fig. 2B). En conclusion, ces résultats soulignent le potentiel extrême des cellules T CD4+CD25+ spécifiques de DBY pour promouvoir le chimérisme hématopoïétique mixte.

Compte tenu du nombre extrêmement bas des DBY-Tregs requis, la question de leur prolifération parallèle à la prise de greffe des MO mâles s'est posée. Effectivement, une expansion de 10-15 fois des DBY-Tregs a été observée dans la rate au jour 10 (Fig. 2C). Ceci est en accord avec leur forte capacité proliférative observée contre les splénocytes mâles *in vivo* (**Fig. 1D**). De manière intéressante, les DBY-Tregs retombent à des niveaux plus bas au jour 16 sans compromettre la prise de greffe à long terme des MO au jour 28 (**Fig. 2B**), suggérant que d'autres mécanismes que la suppression active par DBY-Tregs interviennent plus tard.

### Des mécanismes périphériques sont suffisants pour induire le chimérisme mixte.

La transplantation dans un cadre allogénique nécessite souvent un conditionnement menant à la génération par le thymus d'un répertoire de cellules T partiellement ou totalement nouveau, comme principale cause de l'induction de la tolérance. Dans le système selon l'invention, sans myéloablation ni immunosuppression, les cellules T matures périphériques de l'hôte sont la première barrière à franchir. Pour examiner si l'induction de la tolérance initiée par DBY-Tregs implique uniquement des mécanismes périphériques, des MO mâles et des DBY-Tregs ont été transférées ensemble soit dans des souris sauvages, soit dans des souris thymectomisées. Il s'est avéré que les deux groupes montrent un chimérisme comparable dans toutes les souris à deux mois (Fig. **3A**), démontrant que des mécanismes périphériques sont suffisants pour promouvoir le chimérisme dans les souris traitées avec des DBY-Tregs.

Pour déterminer si la moelle osseuse donneuse requiert des lymphocytes donneurs matures pour induire la tolérance, des MO mâles CD3-/-, dépourvu à la fois de lymphocytes T CD25- et CD25+, ont été transplantées dans des souris femelles C45.1 conditionnées avec des DBY-Tregs. Comme montré à la **Fig. 3B**, l'absence de cellules T mâtures donneuses n'a aucune conséquence sur la prise de greffé des MO.

Couplés à l'expansion transitoire de DBY-Tregs, ces résultats incitaient à examiner le devenir des cellules T CD8+ anti-mâle suite à d'un transfert de MO et à la perfusion des Tregs. Pour cela, le modèle de souris Mata-Hari exprimant un TCR transgénique, spécifique du peptide UTY D^{d} restreint WMHHNMDLI (SEQ ID 2) (21) a été utilisé. Les cellules T CD8+ de Mata-Hari ont été transférées et tracées dans des souris femelles congéniques CD45.1, seules, perfusées avec des MO mâles, ou perfusées avec des MO mâles plus DBY-Tregs. Au jour 11 après transfert de MO, la proportion de cellules T CD8+ de Mata-Hari a augmenté dramatiquement, comparée aux cellules non stimulées, atteignant 2.6±0.4% de cellules T CD8+ au jour 11 (**Fig. 4A-B**). Au contraire, en présence de DBY-Tregs, les cellules Mata-Hari représentaient seulement 0.1±0.03% des cellules T CD8+. Même plus tardivement, lorsque les DBY-Tregs étaient difficilement détectables (**Fig. 2C**), les Mata-Hari n'ont jamais excédé 0.1±0.08% des cellules T CD8+ totales au jour 22 (**Fig. 4B**). Ces résultats démontrent que la prolifération et la fonction des cellules T CD8+ spécifiques des antigènes mâles sont altérées au début de l'induction du chimérisme mixte.

### Développement d'un chimérisme mixte à long terme et multi-lignage

Les DBY-Tregs inhibaient effectivement la réponse immune anti-mâle, promouvant la prise de greffe de MO à court terme, mais leur expansion transitoire pouvait être uniquement associée à une suppression de courte durée, compromettant la prise de greffe à plus long terme de CSH et la tolérance donneur-spécifique.

L'effet d'une simple injection de 10⁵ DBY-Tregs correspondant à deux fois la dose seuil déterminée à la Fig. 2B a été comparé avec 5 injections hebdomadaires de 2-5.10⁵ DBY-Tregs, sur l'établissement d'un chimérisme à long terme (Fig. 5A). Les deux protocoles maintiennent des niveaux soutenus et élevés de chimérisme donneur dans les lymphocytes du sang périphérique entre 3 et 4 mois (9,1±1,8 et 12,3±1,0 respectivement), avec une augmentation graduelle à 9 mois (21,8+2,6 et 18,0±1,7 respectivement) et une prise de greffe à long terme systématique à 18 mois pour toutes les souris. Aucune prise de greffe de MO n'a eu lieu avec des doses élevées de 10⁶ B6-Tregs non spécifiques, même préactivées avec des anticorps anti-CD3 (résultats non montrés). Le chimérisme a été obtenu dans tous les compartiments hématopoïétiques (CD4+, CD8+, B220+, NK1.1+ et CD11c+) dans 100% des receveurs, même dans ceux conditionnés avec une dose unique faible de 10⁵ DBY-Tregs (**Fig. 5B**). De manière importante pour le maintien du chimérisme à long terme, la moelle épinière a également été colonisée par des CSH donneuses, comme défini par leur phénotype Lin-Sca+c-Kit ou l'exclusion de Hoechst de la population latérale (résultats non montrés). Des cellules dendritiques donneuses CD11c+ étaient également présentes dans le thymus, ainsi que des thymocytes se développant normalement, comme démontré par les pourcentages CD4+, CD8+ et CD4+CD8+ (**Fig. 5C**). Ce résultat suggère que le répertoire des cellules T. générées dans le thymus receveur peut être éduqué par les cellules hématopoïétiques à la fois d'origine receveuse et donneuse.

### Tolérance robuste envers les antigènes mâles et immunocompétence vis-à-vis des antigènes tiers

En créant un chimérisme à long terme multi-lignage, il était espéré une tolérance robuste envers tous les antigènes présents dans la greffe. Pour confirmer cela, des souris montrant un minimum de 3-5% de chimérisme ont été provoquées avec différentes formulations d'antigène mâle. Des analyses cytotoxiques *in vivo* ont été réalisées, en perfusant 5.10⁶ splénocytes B6 mâles ou femelles, marqués avec deux niveaux de CFSE. Dans ce système, des souris femelles naïves ont été sensibilisées et ont réagi sélectivement à l'encontre des splénocytes mâles au jour 10 après la perfusion, éliminant quasiment toutes les cellules mâles au jour 16, alors qu'aucune cytotoxicité n'a été observée chez les hôtes mâles (Fig. 6A). Comme attendu, les souris chimérisées n'ont montré aucun rejet des cibles mâles durant les 29 jours de l'expérience (Fig. 6B), indiquant que la tolérance périphérique à long terme envers les antigènes mâles avait été atteinte.

Cette tolérance pouvait être due à un processus de régulation actif et soutenu et/ou une altération/suppression des cellules T anti-mâles. Une suppression soutenue par les DBY-Tregs paraissait improbable puisqu'elles étaient difficilement détectables, étant en dessous du seuil de détection par FACS dans le sang et dans tous les organes lymphoïdes primaires ou secondaires testés plusieurs mois après transfert de MO (résultats non montrés). Pour éclaircir ce point, les réponses aux épitopes CD8 et CD4 ont été découplées pour éviter une suppression immune potentielle par les DBY-Tregs résiduelles. Ainsi, des souris on été provoquées avec l'épitope bien caractérisé UTY en présence de l'adjuvant fort IFA. Aucune réponse anti-mâle n'a été détectée, comme démontré par l'absence de réponse IFNγ peptide-spécifique dans un test ELISPOT (**Fig. 6C**).

Pour exclure la possibilité d'un état général de non réponse dans les chimères, la réponse immune vis-à-vis d'un antigène tiers, absent de la greffe initiale, a été examinée. Après immunisation avec OVA, la fréquence des cellules T productrices de IFNγ antigène-spécifiques et les titres des anticorps IgG OVA-spécifiques ont été suivis. Les souris chimériques et contrôles ont répondu de manière égale à cet antigène (**Fig. 7A**). Enfm, il a été déterminé si la tolérance spécifique des antigènes mâles affectait le rejet de splénocytes mâles chargés avec le peptide OVA257. An utilisant des expériences de cytotoxicité *in vivo,* il a été trouvé que les splénocytes mâles porteurs du peptide étaient aussi susceptibles à la lyse CTL que les cellules femelles porteuses du peptide (**Fig. 7B****, C**). De manière importante, malgré une forte cytotoxicité contre les cellules mâles porteurs d'antigènes tiers, le chimérisme à long terme n'était pas affecté (résultats non montrés).

En conclusion, une tolérance robuste envers tous les antigènes initialement présents dans la greffe, tels que la protéine UTY, a été observée, mais pas envers les antigènes tiers introduits ultérieurement. De plus, cette tolérance n'affecte pas l'immunité normale dirigée contre les cellules cibles donneuses.

### Applications à la prise de greffe secondaire de tissus d'ingénierie

L'absence de réponses immunitaires observées ici envers tous les antigènes mâles dans des souris chimériques est semblable à la tolérance à long terme transgène-spécifique nécessaire pour les applications de transfert de gène en toute sécurité. Il a donc été décidé d'étendre cette tolérance robuste à des transgènes étrangers en établissant le chimérisme moléculaire correspondant en plus des protéines mâles. Il a été choisi d'introduire la protéine EGFP (Enhanced Green Fluorescent Protein), et comme sources de MO modifiées pour exprimer la protéine EGFP (ACTB-EGFP), des souris transgéniques mâles C57BL/6-TgN. Comme montré précédemment avec des expériences de greffe de peau (**8**), EGFP et certainement d'autres antigènes mineurs associés précipitent le rejet des cellules MO mâles EGFP^{high} dans les hôtes mâles (**Fig. 8A**). Comme attendu, le rejet de toutes les cellules MO mâles EGFP a eu lieu dans les femelles non traitées, probablement en raison d'une forte réponse anti-mâle (**Fig. 8A**). Au contraire, des femelles conditionnées avec 10⁵ DBY-Tregs ont montré un chimérisme stable, jusqu'à 10% à 5 mois, avec une forte expression du transgène EGFP (**Fig. 8A****, B**). Ces résultats démontrent qu'une simple perfusion de DBY-Tregs dirigées contre un unique épitope MHC de classe II de DBY est suffisante pour induire une tolérance à long terme envers tous les autres antigènes mineurs présents dans la greffe.

Enfin, il a été déterminé si la forte tolérance établie envers tous les antigènes donneurs présents dans la première greffe pouvait s'exercer en l'absence d'expression de DBY. Pour cela, des souris femelles B6 chimérisées avec une moelle osseuse mâle EGFP (CD45.2+) et DBY-Tregs depuis 5 mois (**Fig. 8A****, B**) ont été transplantées une seconde fois avec des cellules MO mâles ou femelles EGFP, portant un marqueur CD45.1 traçable. Il a été trouvé que les cellules CD45.1+ EGFP^{high} d'origine mâle ou femelle étaient greffées de manière équivalente, montrant que la tolérance s'appliquait à tous les antigènes mineurs, même en l'absence de coexpression de DBY (**Fig. 8C**, **D**).

Pour étendre ces résultats au cadre stringent de la transplantation d'organes, des greffes de peau ont été utilisées comme greffes secondaires. Des souris femelles naïves B6 ont rejeté la greffe de peau EGFP rapidement entre les jours 12 et 16 (**Fig. 8E**). Au contraire, 100% des souris femelles B6 chimérisées avec des cellules MO EGFP mâles ont toléré leur implant, acceptant la greffe de peau indéfiniment (n=8). De manière importante, des souris contrôles chimérisées avec des MO mâles CD45.2, qui n'ont pas été rendues tolérantes envers EGFP, n'ont pas accepté cette allogreffe d'un EGFP tiers (5/6), illustrant la force et la spécificité de la tolérance créée envers le transgène EGFP.

### Greffe de cellules souches hématopoïétiques purifiées sans pré-conditionnement de l'hôte.

Nous avons choisi de purifier les cellules souches hématopoietiques sur leur capacité à exclure le Hoechst. Seules 5 de ces cellules, dites side-population (SP) et dont le phénotype est Lin- ckit+ Thyl.1low Sca-1+ Flk2- CD34- peuvent reconstituer efficacement des hôtes irradiés. Ici, les cellules SP triées à partir de quatre souris CD45.2, sur leur capacité à exclure le Hoechst (Fig 9A), ont été injectées par voie intraveineuse dans des souris congéniques CD45.1 en absence de tout conditionnement myéloablatif. Alors que 4 semaines après la transplantation, moins de 0.5% de chimérisme est observé dans le sang périphérique (Fig. 9B), ce pourcentage augmente rapidement pour atteindre plus de 2% à 6 semaines. Il est à noter que ce chimérisme est maintenu plus d'un an et dans tous les receveurs (Fig. 9C), et concerne les lignages mono/granulocytaires ainsi que B et T (Fig. 9D). Nous avons également fait varier la dose de cellules SP injectées et trouvé qu'un chimérisme maximal pouvait être atteint avec 2.10⁴ cellules SP, purifiées à partir de 2 donneurs (Fig. 9E). Injecter 1.10⁴ cellules SP, purifiées à partir d'une seule moelle donneuse, conduit à l'établissement d'un chimérisme dans toutes les souris mais à avec des pourcentages de greffe variables, révélant que des niches hématopoïétiques vides semblent encore exister. La greffe multi-lignages est à peu près identique à toutes ces différentes doses (Fig. 9F).

### Des cellules souches hématopoïétiques non appariées sur des antigènes mineurs peuvent greffer en présence de Tregs spécifiques

Comme attendu dans le système de rejet mâle/femelle, des CSH mâle transplantées dans des souris femelles sont rejetées, par opposition à la combinaison compatible avec des CSH femelles (Fig. 10A). En revanche, le simple conditionnement des souris receveuses par des DBY-Tregs suffit à inhiber ce rejet, conduit au même niveau de greffe que les souris contrôles et à l'établissement d'un chimèrisme hématopoïétique sur plus d'un an (Fig. 10B-C). Cette immunosuppression est totalement spécifique puisque le même nombre de Tregs non-spécifiques de l'antigène mâle sont incapables d'engendrer un tel effet, et dépend des propriétés régulatrices des cellules T injectées puisque des CD4+CD25- purifiées à partir de la même souris Marilyn sont inefficaces (Données non montrées). Il est ainsi démontré que les résultats obtenus avec la moelle osseuse peuvent être reproduits à partir de CSH purifiées.

En résumé, il a été démontré qu'un conditionnement minimal avec un petit nombre de CD4+CD25+ dirigées contre un seul épitope de DBY favorise la prise de greffe de cellules MO et établit une tolérance antigène-spécifique, vis-à-vis de multiples antigènes mineurs histocompatibles chez des adultes haploidentiques. De manière intéressante, une greffe secondaire portant un de ces antigènes mineurs, tel que le transgène EGFP, persiste, que l'antigène d'origine ayant induit la tolérance soit présent ou non. Cela ouvre des perspectives pour l'application thérapeutique du chimérisme mixte pour conditionner l'hôte pour le greffage ultérieur de tissus ou pour le transfert de gènes (**6**). Des Tregs mono-spécifiques peuvent maintenant être multipliées *in vitro* (**19**) et la génération *de novo* de Tregs spécifiques d'antigènes a récemment été obtenue chez l'homme (**20**). Avec l'arrivée de tels protocoles, les Tregs mono-spécifiques pourraient fournir de nouvelles opportunités pour remplacer les traitements immunodépressifs myéloablatifs et potentiellement mortels pour la transplantation de MO ou de CSH autologues modifiées génétiquement avant le transfert de gènes.

### REFERENCES

- 1.: Bessis, N. et al. Gene Ther 11, Suppl 1:S10-17. (2004).
- 2.: Jooss, K. et al. Gene Ther 10, 955-963. (2003).
- 3.: Brown, B. D. et al. Blood 100, 1133-1140. (2002).
- 4.: Herzog, R. W. et al. Mol Ther 4, 192-200. (2001).
- 5.: Sarukhan, A. et al. J Virol 75, 269-277. (2001).
- 6.: Sykes, M. Immunity 14, 417-24. (2001).
- 7.: Nikolic, B. et al. Diabetes 53, 376-83. (2004).
- 8.: Andersson, G. et al. Mol Ther 8, 385-91. (2003).
- 9.: Wekerle, T. et al. Nat Med 6, 464-9. (2000).
- 10.: Lin, C.Y. et al. Nat Immitnol 3, 1208-13. (2002).
- 11.: Simpson, E. et al. Eur J Immunogenet 28, 505-13 (2001).
- 12.: Waldmann, H. & Cobbold, S. Science 305, 209-12. (2004).
- 13.: Walsh, P.T et al. J Clin Invest 114, 1398-403. (2004).
- 14.: Taylor, P.A. et al. Blond 104, 3804-12. (2004).
- 15.: Trenado, A. et al. J Clin Invest 112, 1688-96. (2003).
- 16.: Joffre, O. et al. Blood 103, 4216-21. (2004).
- 17.: Gross, D.A. et al. Bloom 102, 4326-8. (2003).
- 18.: Thery, C. et al. Nat Immunol 3, 1156-62..(2002).
- 19.: Tarbell, K.V. et al. J Exp Med 199, 1467-77. (2004).
- 20.: Walker, M.R. et al. Proc Natl Acad Sci U S A (2005).
- 21.: Valujskikh, A. et al. Nat Immunol 3, 844-51. (2002).

## Revendications

1. composition pharmaceutique comprenant :
- des cellules T régulatrices CD4+ CD25+ spécifiques d'un ou plusieurs antigènes mineurs d'histocompatibilité; et
- des cellules souches avantageusement hématopoïétiques, porteuses d'au moins ledit antigène qui ne sont pas des cellules souches directement issues d'embryons humains.

2. Composition pharmaceutique selon la revendication 1 *caractérisée* en ce en ce que les cellules T régulatrices CD4+ CD25+ sont monospécifiques.

3. Composition pharmaceutique selon la revendication 1 ou 2 ***caractérisée* en ce que** les cellules T régulatrices CD4+ CD25+ et/ou les cellules souches sont autologues.

4. Composition pharmaceutique selon l'une des revendications précédentes ***caractérisée* en ce que** l'antigène mineur est un antigène mineur mâle, préférentiellement choisi dans le groupe comprenant DBY, UTY et SMCY.

5. Composition pharmaceutique selon l'une des revendications précédentes ***caractérisée* en ce que** les cellules souches sont transduites par un vecteur porteur d'un gène codant l'antigène mineur.

6. Composition pharmaceutique selon l'une des revendications précédentes ***caractérisée* en ce que** les cellules souches portent au moins un transgène, avantageusement introduit à l'aide d'un vecteur viral.

7. Composition pharmaceutique selon l'une des revendications précédentes comme composition de combinaison pour une utilisation simultanée, séparée ou étalée dans le temps en thérapie cellulaire ou génique.

8. Utilisation d'une composition phannaceutique selon l'une des revendications 1 à 7 pour la préparation d'un médicament destiné à augmenter la tolérance immunitaire chez un hôte histocompatible.

9. Utilisation selon la revendication 8 ***caractérisée* en ce que** la tolérance immunitaire est augmentée en vue de l'expression stable du transgène.

10. Utilisation selon la revendication 8 ou 9 ***caractérisée* en ce que** la tolérance est augmentée en vue de la transplantation d'un tissu issu d'un donneur porteur d'au moins ledit antigène.

11. Utilisation selon la revendication 10 ***caractérisée* en ce que** la transplantation du tissu est une greffe de peau.

## Claims

1. Pharmaceutical composition comprising:
- CD4+ CD25+ regulatory T cells specific for one or more minor histocompatibility antigens; and
- stem cells which are not stem cells directly obtained from human embryos, advantageously haematopoietic, carrying at least the said antigen.

2. Pharmaceutical composition according to Claim 1, ***characterized* in that** the CD4+ CD25+ regulatory T cells are monospecific.

3. Pharmaceutical composition according to Claims 1 or 2, ***characterized* in that** the CD4+ CD25+ regulatory T cells and/or the stem cells are autologous.

4. Pharmaceutical composition according to one of the preceding claims, ***characterized* in that** the minor antigen is a male minor antigen, preferably selected from the group comprising DBY, UTY and SMCY.

5. Pharmaceutical composition according to one of the preceding claims, ***characterized* in that** the stem cells are transduced by a vector carrying a gene coding for the minor antigen.

6. Pharmaceutical composition according to one of the preceding claims, ***characterized* in that** the stem cells carry at least one transgene, advantageously introduced by means of a viral vector.

7. Pharmaceutical composition according to one of the preceding claims, as a combination composition for simultaneous, separate or time-spread use in cell or gene therapy.

8. Use of a pharmaceutical composition according to one of Claims 1 to 7, for preparing a medicament for increasing the immune tolerance of a histocompatible host.

9. Use according to Claim 8, ***characterized* in that** the immune tolerance is increased for the stable expression of the transgene.

10. Use according to either of Claims 8 and 9, ***characterized* in that** the tolerance is increased for the transplantation of a tissue from a donor carrying at least the said antigen.

11. Use according to Claim 10, ***characterized* in that** the tissue transplantation is a skin graft.

## Patentansprüche

1. Pharmazeutische Zusammensetzung, Folgendes umfassend:
- spezifische CD4⁺ CD25⁺-regulatorische T-Zellen eines Minorhistokompatibilitätsantigens oder mehrerer Minorhistokompatibilitätsantigene; und
- Stammzellen, die keine Stammzellen sind, die direkt aus menschlichen Embryonen stammen, vorteilhafter Weise hämatopoetische Stammzellen, wobei die Stammzellen Träger mindestens dieses Antigens sind.

2. Pharmazeutische Zusammensetzung nach Anspruch 1, **dadurch gekennzeichnet, dass** die spezifischen CD4⁺ CD25⁺-regulatorischen T-Zellen monospezifisch sind.

3. Pharmazeutische Zusammensetzung nach Anspruch 1 oder 2, **dadurch gekennzeichnet, dass** die spezifischen C04 CD25⁺-regulatorischen T-Zellen und/oder die Stammzellen autolog sind.

4. Pharmazeutische Zusammensetzung nach einem der vorhergehenden Ansprüche, **dadurch gekennzeichnet, dass** das Minorantigen ein männliches Minorantigen ist, das bevorzugt aus der Gruppe ausgewählt ist, die DBY, UTY und SMCY umfasst.

5. Pharmazeutische Zusammensetzung nach einem der vorhergehenden Ansprüche, **dadurch gekennzeichnet, dass** die Stammzellen durch einen Vektor transduziert werden, der Träger eines das Minorantigen kodierenden Gens ist.

6. Pharmazeutische Zusammensetzung nach einem der vorhergehenden Ansprüche, **dadurch gekennzeichnet, dass** die Stammzellen mindestens ein Transgen tragen, das vorteilhafter Weise mittels eines viralen Vektors eingeschleust wird.

7. Pharmazeutische Zusammensetzung nach einem der vorhergehenden Ansprüche als Kombinationszusammensetzung für eine gleichzeitige, getrennte oder über die Zeit verteilte Verwendung in einer Zell- oder Gentherapie.

8. Verwendung einer pharmazeutischen Zusammensetzung nach einem der Ansprüche 1 bis 7 zur Herstellung eines Medikaments, das dazu bestimmt ist, die Immuntoleranz bei einem histokompatiblen Wirt zu erhöhen.

9. Verwendung nach Anspruch 8, **dadurch gekennzeichnet, dass** die Immuntoleranz im Hinblick auf die stabile Expression des Transgens erhöht wird.

10. Verwendung nach Anspruch 8 oder 9, **dadurch gekennzeichnet, dass** die Toleranz im Hinblick auf die Transplantation eines Gewebes erhöht wird, das von einem Spender stammt, der Träger zumindest des Antigens ist.

11. Verwendung nach Anspruch 19, **dadurch gekennzeichnet, dass** die Transplantation des Gewebes eine Hautverpflanzung ist.
